(19) **Europäisches Patentamt / European Patent Office / Office européen des brevets**

(11) **EP 3 494 910 A1**

(12) # EUROPEAN PATENT APPLICATION
published in accordance with Art. 153(4) EPC

(43) Date of publication:
**12.06.2019 Bulletin 2019/24**

(21) Application number: **17836288.5**

(22) Date of filing: **20.07.2017**

(51) Int Cl.:
**A61B 17/34** (2006.01)

(86) International application number:
**PCT/CN2017/093605**

(87) International publication number:
**WO 2018/024106 (08.02.2018 Gazette 2018/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **02.08.2016 CN 201610626424**

(71) Applicant: **5R Med Technology (Chengdu) Co., Ltd Chengdu, Sichuan 610000 (CN)**

(72) Inventor: **ZHU, Moshu**
**Chengdu**
**Sichuan 610000 (CN)**

(74) Representative: **Gong, Jinping**
**CocreateIP**
**Eggenfeldenerstraße 56**
**81929 München (DE)**

(54) **PUNCTURE DEVICE SEALING MEMBRANE COMPRISING REVERSE GROOVES**

(57) The invention discloses a trocar seal membrane with reverse Concave-channels. Said seal membrane comprises a proximal opening proximal opening, a distal aperture, and a sealing wall extending from the distal aperture to the proximal opening, said distal aperture formed by a sealing lip for accommodating the inserted instrument and forming a seal housing; said sealing lip comprises a longitudinal axis and a transverse plane substantially perpendicular to said longitudinal axis. Said sealing wall in the lip-adjacent area comprises the main rotary-wall and a plurality of reverse Concave-channels, which are recessed from the distal surface of the main rotary-wall toward the proximal surface and the reverse Concave-channel opening pointing to the direction of the distal surface. The shape of the reverse Concave-channel from the perspective of proximal surface is represented as a hollow convex-rib that is raised from the proximal surface. Said reverse Concave-channel has the functions of enlarging hoop circumference, reducing the wrapped area, improving lubrication reliability, increasing the axial tensile stiffness, thereby greatly reducing said frictional resistance and the stick-slip.

Fig. 9

**Description**

**TECHNICAL FIELD**

The present invention relates to a minimally invasive surgical instrument, and in particular, to a trocar sealing element.

**BACKGROUD OF THE PRESENT INVENTION**

A trocar is a surgical instrument, that is used to establish an artificial access in minimally invasive surgery (especially in rigid endoscopy). Trocars comprise in general a cannula and an obturator. The surgical use of trocars generally known as: first make the initial skin incision at the trocar insertion site, then insert the obturator into the cannula, and then together they facilitated penetration of the abdominal wall through incision into the body cavity. Once penetrated into the body cavity, the obturator is removed, and the cannula will be left as access for the instrument get in/out of the body cavity.

In rigid endoscopy surgery, it is usually necessary to establish and maintain a stable pneumoperitoneum for the sufficient surgical operation space. The cannula comprises a sleeve, an outer body, a seal membrane (also known as instrument seal) and a duck bill (also known as closure valve). Said cannula providing a channel for the instrumentation in/out of the body cavity, said outer body connecting the sleeve, the duck bill and the seal membrane into a sealing system; said duck bill normally not providing sealing for the inserted instrument, but automatically closing and forming a seal when the instrument is removed; said seal membrane accomplishing a gas-tight seal against the instrument when it is inserted.

In a typical endoscopic procedure, it is usually set up 4 trocars(access), i.e. 2 sets of small diameter cannula (normally 5mm in diameter), and 2 sets of large diameter cannula (normally 10~12mm in diameter). Instruments, in general passing through a small cannula are only for ancillary works; herein one large cannula as an endoscope channel, and the other large cannula as the main channel for surgeon to perform surgical procedures. Through said main channel thereof, 5mm diameter instruments used in approximately 80% of the procedure, and said large cannula used in approximately 20% of the procedure; furthermore, 5mm instruments and large diameter instruments need to be switched frequently. The small instruments are mostly used, so that the sealing reliability of which is more important. The large instruments are more preferably used in a critical stage of surgery (Such as vascular closure and tissue suturing), therein switching convenience and operational comfort are more important.

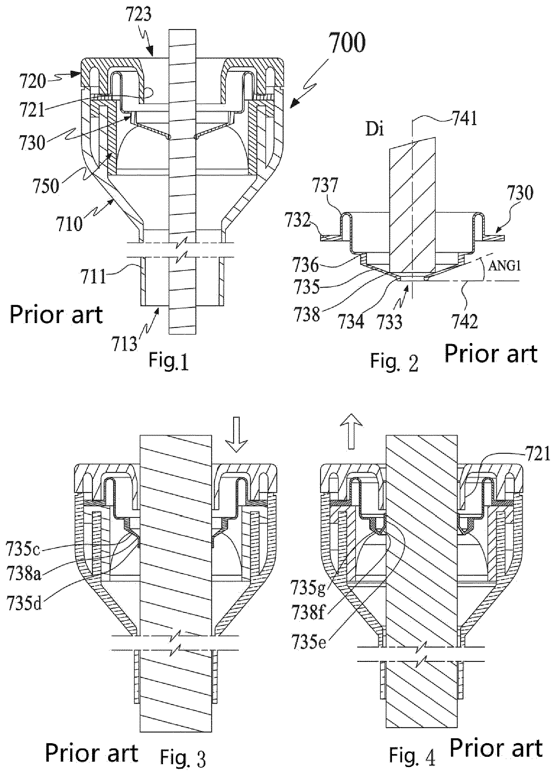

FIG. 1 and FIG. 2 depict a typical 12mm diameter cannula 700. Said cannula 700 comprises a lower housing 710, an upper housing 720, a seal membrane 730 which sandwiched between the lower housing 710 and the upper housing 720, and a duckbill seal 750. Said lower housing 710 including center hole 713 defined by an elongated tube 711. Said upper housing 720 including the proximal hole 723 defined by the inner wall 721. Said membrane 730 including a proximal opening 732, a distal aperture 733, a sealing lip 734, a frustum sealing wall 735, a flange 736 and an outer floating portion 737. Said distal opening 733 formed by a sealing lip 734. Said sealing lip 734 defining a longitudinal axis 741, transverse plane 742 substantially perpendicular to said axis 741; define the angle between the rotary-generating line (or generatrix) of the frustum sealing wall 735 and the transverse plane 742 as a guide angle ANG1.

As illustrated in FIG.1, when a 5mm diameter instrument inserted, it is approximately considered that only hoop force generated by the deformation of the sealing lip 734, ensures a reliable seal for the instrument. It is nevertheless favorable to operate the instrument from various extreme angles in surgery. There's a lot space left for the 5mm-instrument to move radially in the 12mm diameter cannula, so that greater radial force would be taken by the sealing lip 734. Therefore, the sealing lip 734 should have sufficient hoop force for the inserted 5mm diameter instrument to ensure its sealing reliability thereof.

As illustrated in FIG.2, drawing a cylinder of Di (Di>5mm) to cut the sealing wall 735 forms an intersecting line 738. It is easy to understand for those skilled in the art, when an Di diameter instrument is inserted, the strain (stress) of said sealing wall 735 in the area from the sealing lip 734 to the intersecting line 738 will be larger, so the area refer to as lip-adjacent area(or concentration stress area). While the strain (stress) of said sealing wall 735 from the intersecting line 738 to the flange 736 is small. However, the different diameter (Di value) makes the boundary range of the lip-adjacent area(or concentration stress area) change larger or smaller. For the convenience of quantification, it is defined when Di is designed as the maximum diameter of the surgical instrument passing through the seal membrane, the area from the sealing lip 734 to the intersection line 738 is the lip-adjacent area.

As illustrated in FIG.3, when a large diameter instrument is inserted (e.g. 12.8 mm), the sealing lip 734 will expand to a suitable size to accommodate the inserted instrument; said sealing wall 735 is divided into two portions: a conical wall 735c and a cylindrical wall 735d; said cylindrical wall 735d wrapped around the outer surface of the instrument to form a wrapped area with a high concentration of stress. Defining the intersecting line of the conical wall 735c and the cylindrical wall 735d as intersecting line 738a. When the instrument is removed, said sealing wall 735 return to natural state, and said intersecting line 738a spring-back to a ring radius of Dx, defined as intersecting line 738b, (not shown in FIG); said intersecting line 738b is a bending boundary line when inserting a large diameter instrument. The angle between the rotary generating line of said conical wall 735c and the transverse plane 742 defines as ANG2, ANG2>ANG1; that is, when the large-diameter instrument is inserted, said sealing wall 735 rotates and stretch around its intersection line of said flange 736. Defining the height of the cylindrical wall 735d as Ha, not a fixed value; the factors such as different size of said distal aperture , different size of said sealing lip, different thickness of said sealing wall, different said guide angle or different diameter of inserted instrument, make Ha different.

The instrument inserted into the sealing membrane and moved during surgical procedure, there is large frictional resistance between the wrapped area and the inserted instrument. Said large frictional resistance is normally easy to cause the seal inversion, poor comfort of performance, fatigue performance, even result in cannula insecurely fixed on the patient's abdominal wall etc., so that the performance of cannula is affected.

Among the defects caused by the large frictional resistance, the seal inversion is one of the most serious problems that affecting the performance of the cannula. As illustrated in FIG. 4, when a large diameter instrument is removed, easily cause seal inversion. When inversion happened, said sealing wall 735 divided into a cylindrical wall 735e, a conical wall 735f, and a conical wall 735g; said cylindrical wall 735e wrapped around the outer surface of the instrument to form a wrapped area with a high concentration of stress. Defining the height of the cylindrical wall 735e to be Hb, normally Hb>Ha; that is, the frictional resistance when the instrument is removed greater than it when the instrument is inserted, this difference affects the surgeon's operating feeling and even make the surgeon confused. More seriously, the inversion of the seal membrane may stretch into the proximal hole 723, that is the seal membrane positioned between the instrument and the inner wall 721 gets completely jammed. Measures for preventing the seal inversion are respectively disclosed in US7112185 and US7591802, and those measures can effectively reduce the probability of inversion but not completely solve the problem.

The simplest way to reduce the frictional resistance is reducing the coefficient of friction between the two contacting surfaces with grease, but the reliability of this way is not good. During procedures, due to instruments long-term repeated scraping with the seal membrane and repeated switching, it is easy to erase the grease off and carried away, resulting in bad lubrication.

A protector assembly adjoined by a seal membrane is disclosed in US 5342315. Said protector to permit the sharp edge of the instrument to pass through the opening in the seal membrane without causing damage to the seal membrane, and the surface friction coefficient of the protector assembly is smaller than the surface friction coefficient of the seal membrane, which results in frictional resistance in a certain degree, but the lip-adjacent area is normally not completely covered by the protector assembly.

A seal member with ribs (or projections) is disclosed in US5827228, that is a plurality of spaced ribs provided to extend outwardly from center hole to reduce surface contact between the inserted instrument and the seal member, and thereby reducing the frictional resistance, a similar ribs which disclosed in EP0994740 also reducing surface contact and strengthen the tensile of the seal member oriented to axial.

A sealing element comprising a flexible wall closed annularly with the edges foldable in a wave-like manner is disclosed in US 7842014, wherein the wall bears a wave-like sealing lip and are a wavy pleated seal body, in such manner it can enlarge hoop circumference , and reduce the hoop force to a certain extent.

Chinese invention application CN101480354A (currently rejected) discloses a seal member containing an easily deformable groove, wherein is characterized in that it has a plurality of easily deformable grooves on the conical surface of the seal member from the sealing lip; said the thickness of the deformable groove wall is much smaller than the thickness of the conical surface wall, primary take advantage of the elongation of the deformable groove to accommodate the inserted large diameter instrument.

Although, in the prior art many solutions for reducing the frictional resistance have been disclosed, these solutions basically only propose measures from one certain factor affecting frictional resistance, the effect of which is small or not obvious. Some modifications solved a certain defects may lead to cause another bug. Such as, reinforcing ribs on the seal membrane to reduce surface contact, meanwhile strengthen the tensile of the seal membrane; or a deformable groove with a thickness much smaller than that of a truncated conical surface can cause the deformable groove to be easily damaged; due to the adoption of said wave-like sealing lip which enlarge hoop circumference, the sealing reliability will be sacrificed when a 5mm diameter instrument is inserted, if the wave-like sealing lip is used but without enlarge hoop circumference, the wave-like sealing lip will lose its improvement effect. In summary there are many factors affecting the frictional resistance, and the comprehensive effects of various factors must be considered in the perspective of mechanics and tribology.

The seal membrane is preferably produced from rubber such as natural rubber, silicone or polyisoprene, its mechanical properties including super elastic and viscoelastic. Although the mechanical model of the rubber deformation process is complicated, it can still apply the generalized Hooke's law to describe approximatively its elastic behavior; and Newton's internal friction law to describe the viscous behavior. Research suggests that the main factors affecting the friction of the two surfaces in contact between the rubber and the instrument include: the smaller the friction coefficient of said two surfaces, the smaller the friction is; the better lubrication condition of said two surfaces in contact, the friction smaller is; the smaller normal pressure of said two surfaces, the friction smaller is. Comprehensively considering the above factors, the present invention proposes better solutions for reducing the frictional resistance between the seal membrane and the inserted instrument.

In addition to said frictional resistance greatly affecting the performance of the cannula assembly, the stick-slip of the seal membrane is another main factor affecting the performance of trocar. Said stick-slip means that when the instrument moves longitudinally in the sleeve, the sealing lip and lip-adjacent area sometimes are relatively statically attached to the instrument (at this point, the friction between the instrument and the seal membrane is mainly static friction.); but sometimes it produced a relatively slippery phenomenon with the instrument (at this point, the friction between the instrument and the seal membrane is mainly dynamic friction.); and said static friction is much greater than said dynamic friction. The two frictions alternately occur, which causes the movement resistance and speed of the instrument in the seal membrane to be unstable. It is easy to be understand for those skilled in the art, that in minimally invasive surgery the surgeon can only use surgical instruments to touch (feel) the patient's organs and observe a part of the working head of the instruments through endoscopic image system. In this case where the vision is limited and it cannot be touched, the surgeon typically uses the feedback of the resistance when moving instruments as one of the information to judge whether the operation is abnormal nor not. The stick-slip affects the comfort of operation, the accuracy of positioning, and even induces the surgeon to make false judgment.

During the surgical application of the cannula, the stick-slip is difficult to avoid, but can be reduced. Researches have shown that said stick-slip is affected by two main factors: one is that the smaller the difference between the maximum static friction and the dynamic friction, the weaker the stick-slip is; the other is that the larger the axial tensile stiffness of the seal membrane, the weaker the stick-slip is. Avoiding excessive the hoop force between the seal membrane and the instrument, reducing the two surfaces contacted, maintaining good lubrication, respectively, can reduce the difference between the maximum static friction and the dynamic friction, thereby reducing stick-slip, meanwhile, increasing the axial tensile stiffness of the seal membrane also helps to reduce the stick-slip phenomenon. The invention also proposes measures for improving stick-slip.

In summary, so far, there is no cannula that can effectively solve the said problems.

## SUMMARY OF PRESENT INVENTION

In conclusion, one object of the invention is to provide a trocar seal membrane, said seal membrane comprises a proximal opening, a distal aperture, and a sealing wall from the distal aperture extending to the proximal opening, said distal aperture formed by a sealing lip for accommodating the inserted instrument and forming a gas-tight seal. Said the sealing wall includes a proximal surface and a distal surface. Said seal membrane can ensure a reliable seal for the inserted 5mm instrument, and reduce frictional resistance and improve stick-slip when a large-diameter instrument is inserted.

As described in the background, the wrapped area formed by the sealing lip and the lip-adjacent area when a large diameter instrument inserted, is the major factor cause of frictional resistance. For reducing said frictional resistance, comprehensive consideration should be given such as reducing the radial stress between the instrument and the seal membrane, reducing said wrapped area, and reducing the actual contact area of the two surfaces. It is easy to understand for those skilled in the art that in accordance with the generalized Hooke's law and Poisson effect, enlarge hoop circumference, and reduce hoop strain (stress), thereby reducing radial strain (stress). But it should be noted that it is impossible to enlarging the hoop circumference in order to reduce the strain of the sealing lip which will result in reduced sealing reliability when applying 5mm instruments. Since the stress in the lip-adjacent area is highly concentrated when applying a large diameter instrument, the hoop circumference of the lip-adjacent area should be rapidly increased. In regard to outside the lip-adjacent area, since the strain (stress) is small, it is not necessary to adopt measures to enlarge the hoop circumference. In addition, enlarging the hoop circumference, in the meantime increasing the axial tensile stiffness in the lip-adjacent area and maintain good lubrication (reducing difference between the maximum static friction and dynamic friction), thereby the stick slip in the lip-adjacent area is improved.

In one aspect of the present invention, said seal membrane comprises a proximal opening, a distal aperture, and a sealing wall from the distal aperture extending to the proximal opening, said distal aperture formed by a sealing lip for accommodating the inserted instrument forms a gas-tight seal, said sealing wall includes a proximal surface and a distal surface. Said sealing lip comprises a longitudinal axis and a transverse plane substantially perpendicular to said axis. Said sealing wall in the lip-adjacent area, comprises the main rotary-wall and a plurality of reverse Concave-channel, which is recessed from the distal surface of the main rotary-wall toward the proximal surface and the reverse Concave-channel opening oriented to the distal surface. The shape of the reverse Concave-channel from the perspective of proximal surface is represented as a hollow convex-rib that is raised from the proximal surface. In the lip-adjacent area, each of the reverse Concave-channel comprises two side sealing-walls, which are areas defined by the two edges extending laterally outward from the sealing lip and this region with a gradually increasing width. The sealing lip is annular, and the cross section of the reverse Concave-channel is approximately U-shaped. Said seal membrane includes a flange at which the main rotary-wall extendedly intersects, or simultaneously the main rotary-wall and the reverse Concave-channel extends and intersect, and an outer floating portion including at least one lateral pleat extending from the flange to the proximal opening.

Optionally, in one embodiment, this geometric relationship, herein is the angle between the two edges of the side sealing-wall of reverse Concave-channel in the lip-adjacent area, conforms to the following equation:

$$\theta \geq \arctan \frac{9\pi R(R_i - R)\cos \alpha}{P(R_i + 9R)(R - R_0)}$$

and $\alpha + \theta \leq 90°$
where:

θ= the angel between two edges of the reverse Concave-channel side sealing-wall in the lip-adjacent area;
α=The angle between the rotary generating line and the transverse plane in the lip-adjacent area;
arctan= arctangent function;
cos=Cosine function;
π=Circumference ratio;
R=Radius;
Ri=the largest radius designed for the surgical instrument passed through the seal membrane;
R0=Radius of the sealing lip;
P=Number of reverse Concave-channel.

Outside the lip-adjacent area, the width of the side sealing-wall does not conform to the above equation, the side sealing-wall is gradually smaller. This, such as it is, can simplify mold processing and increase production efficiency of the seal membrane, and help to reduce actual contact area of the two surfaces between the instrument and the seal membrane.

In one aspect of the invention, said seal membrane comprises a proximal opening, a distal aperture, and a sealing wall from the distal aperture extending to the proximal opening, said distal aperture formed by a sealing lip for accommodating the inserted instrument forms a gas-tight seal, said sealing wall comprising a proximal surface and a distal surface, said sealing lip comprising a longitudinal axis and a transverse plane substantially perpendicular to said longitudinal axis. Said sealing wall in the lip-adjacent area, comprises the main rotary-wall and a plurality of reverse Concave-channels, which are recessed from the distal surface of the main rotary-wall toward the proximal surface and the reverse Concave-channel opening oriented to the distal surface, the shape of the reverse Concave-channel from the perspective of proximal surface is represented as a hollow convex-rib that is raised from the proximal surface. In the lip -adjacent area, each of the reverse Concave-channels comprises two side sealing-walls, which are areas defined by the two edges extending laterally outward from the sealing lip with a gradually increasing width. The sealing lip is cylindrical, and the cross section of the reverse Concave-channel is approximately V-shaped.

In another aspect of the invention, said seal membrane comprises a proximal opening, a distal aperture, and a sealing wall from the distal aperture extending to the proximal opening, said distal aperture formed by a sealing lip for accommodating the inserted instrument and forming a gas-tight seal, said sealing wall comprising a proximal surface and a distal surface. Said sealing lip comprises a longitudinal axis and a transverse plane substantially perpendicular to said longitudinal axis. Said sealing wall in the sealing lip-adjacent area, comprises the main rotary-wall and a plurality of reverse Concave-channels, which are recessed from the distal surface of the main rotary-wall toward the proximal surface and the reverse Concave-channels opening oriented to the distal surface, while the reverse Concave-channel from the perspective of proximal surface is represented as a hollow convex-rib that is raised from the proximal surface. In the sealing lip-adjacent area, each of the reverse Concave-channels comprises two side sealing-walls, which are areas defined by the two edges extending laterally outward from the sealing lip and this region with a gradually increasing width. The cross section of the reverse Concave-channel is approximately U-shaped, and said the thickness of the reverse Concave-channel side sealing-wall is much smaller than the thickness of the main rotary-wall.

The other object of the invention is to provide a trocar seal assembly. Said seal includes a lower retainer ring, a seal membrane, a protect device, an upper retainer ring, an upper body, an upper cover; said seal membrane and said protector device are sandwiched between the upper retainer ring and the lower retainer ring, said protect device used to protect the seal membrane from sharp edges of the inserted instrument. Said seal membrane further includes a flange at which the main body wall extendedly intersects, or simultaneously the main rotary-wall and the reverse Concave-channel extended to be intersected, and an outer floating portion including at least one lateral pleat extending from the flange to the proximal opening. The proximal opening of said the seal membrane sandwiched between the upper body and the upper cover, said outer floating portion makes the seal membrane and protector float laterally in the housing formed by the upper body and the cover.

It is believed that the above invention or other objects, features and advantages, will be understood with the drawings and detailed description.

## DESCRIPTION OF THE DRAWINGS

A more complete appreciation of this invention, and many of the attendant advantages thereof will be readily apparent as the same becomes better understood by reference to the following detailed description, where:

FIG. 1: shows a simulated distorted view of the cannula with the 5 mm diameter instrument inserted in the prior art;
FIG. 2: shows a detailed sectional view of the seal membrane730 in the prior art;
FIG. 3: shows a simulated distorted view of the cannula with the 12.8mm diameter instrument inserted in the prior art;
FIG. 4: shows a simulated distorted view of the cannula with the 12.8mm diameter instrument removed in the prior art;
FIG. 5: shows a 3D perspective partial sectional view of the cannula according to the invention;
FIG. 6: shows an exploded view of the seal membrane assembly of the cannula in FIG. 5: FIG. 7: shows a 3D perspective partial sectional view of the seal membrane assembly in FIG. 6;
FIG. 8: shows a 3D perspective view of the seal membrane without the proximal end and floating portion in FIG. 6;
FIG. 9: shows a sectional view along line 9-9 in FIG. 8;
FIG. 10: shows a reversed 3D perspective sectional view of the seal membrane in FIG. 8;
FIG. 11-12: shows a segmentation view of the seal membrane after the circumferential cutting separation in FIG. 8;
FIG. 13: shows a simulated distorted view of the seal membrane assembly with the 12.8mm instrument inserted in FIG.7;
FIG. 14: shows a reverse 3D perspective view of the seal membrane assembly in FIG. 13;
FIG. 15: shows a sectional view along-line 15-15 in FIG. 13
FIG. 16: shows a sectional view along-line 16-16 in FIG. 13
FIG. 17: shows a perspective view of the seal membrane of the second embodiment according to the invention;
FIG. 18: shows a partial view along-line 18-18 in FIG. 17
FIG. 19: shows a sectional view along-line 19-19 in FIG. 17
FIG. 20: shows a reverse 3D perspective view of the seal membrane in FIG. 17;
FIG. 21: shows a perspective view of the seal membrane of the third embodiment according to the invention;
FIG. 22: shows a reverse 3D perspective view of the seal membrane in FIG. 21;
FIG. 23: shows a sectional view along-line 23-23 in FIG. 22;
FIG. 24: shows a sectional view along-line 24-24 in FIG. 22;
FIG. 25: shows a partial view along-line 25-25 in FIG. 22;
FIG. 26: shows a simulated 3D perspective sectional view of the seal membrane in FIG. 21 while inserting the titanium applier;
FIG. 27: shows a simulated perspective sectional view under the function of titanium applier in FIG. 26;

In all views, the same referred number shows the same element or assembly.

## DETAILED DESCRIPTION OF PREFERRED EMBODIMENTS

Embodiments of the invention are disclosed herein, however, it should be understood that the disclosed embodiments are merely examples of the invention, which may be implemented in different ways. Therefore, the invention is not intended to be limited to the detail shown, rather, it is only considered as the basis of the claims and the basis for teaching those skilled in the art how to use the invention.

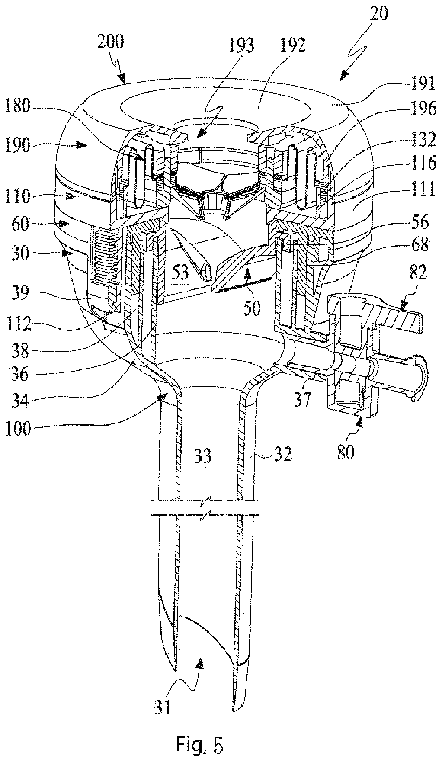

FIG.5 shows an overall view of the structure of trocar. A typical trocar comprises an obturator 10 (not shown) and a cannula 20. The cannula 20 comprises an open proximal end 192 and an open distal end 31. In a typical embodiment, said obturator 10 passes through said cannula 20, together they facilitated penetration of the abdominal wall through incision into the body cavity. Once penetrated into the body cavity, the obturator 10 is removed, and the cannula 20 will be left as access for the instrument get in/out of the body cavity. Said proximal end 192 in the external position of the patient and said distal end 31 in the internal position. A preferred cannula 20 can be divided into the first seal assembly 100 and the second seal assembly 200. Locking receptacle 39 in said seal assembly 100 can be locked with snap-in projection 112 in said seal assembly 200. The cooperation of snap-in projection 112 and the locking receptacle 39 can be quick release by one hand. The main purpose is for convenience of taking out tissues or foreign matter from the patient in the surgery. There are multiple ways to implement the quick release connection of said seal assembly 100 and assembly 200. In addition to the structure shown in this embodiment, a threaded connection, a rotary snap-in or other quick lock structure also may be applied. Alternatively, said assembly 100 and assembly 200 can be designed as a structure that can not be split quickly.

FIG.5 shows the composition and assembly relationship of the first seal assembly 100. The lower body 30 includes an elongated tube 32, which defines the sleeve 33 passed through the distal end 31 and is connected to the outer housing 34. Said lower body 30 comprises an inner wall 36 supporting duck bill seal and a valve bore 37 that communicates with the inner wall 36.The plunger 82 mounted in the valve body 80, the said two are mounted into said valve bore 37. The flange 56 of the duck bill seal 50 is sandwiched between the inner wall 36 and the lower cover 60. There are various ways of fixing between the lower cover 60 and the lower body 30, such as the interference fit, ultrasonic welding, glue bonding, and snap fastening. 4 cylinders 68 of said lower cover 60, in this embodiment, 4 holes 38 of said lower body 30 are adopted to interference fit, so that the duckbill seal 50 is in the compressed state. Said tube 32, said the inner wall 36, said duck bill seal 50, said valve body 80 and said plunger 82 together are comprised the first chamber. Said duck bill seal 50, in this embodiment, is a single-slit, while other types of closure valves may also be used, including flapper valves, multi-silted duck bill valves. When the instrument is passed through said duck bill seal 50, the duckbill 53 will be opened, but it generally does not provide a complete seal against the instrument. When the instrument is removed, said duckbill 53 closed and substantially prevents insufflation fluid from escaping through the first chamber.

FIG.5 shows the composition and assembly relationship of the second seal assembly 200. The seal membrane assembly 180 is sandwiched between the upper cover 110 and the upper body 190. The proximal end 132 of the seal membrane assembly 180 is secured between the inner ring 116 of the upper cover 110 and the inner ring 196 of the upper body 190. There are various secured ways between the upper body 190 and the upper body 110, such as the interference fit, ultrasonic welding, glue bonding, and snap fastening. The connection method, shown in this embodiment, is the outer shell 191 of the upper body 190 and the outer shell 111 of the upper cover 110 are secured by ultrasonic welding, so that the proximal end 132 of the seal membrane assembly 180 is in the compressed state. The center hole 113 of said upper cover 110, said inner ring 116, and said seal membrane assembly 180 together are comprised the second chamber.

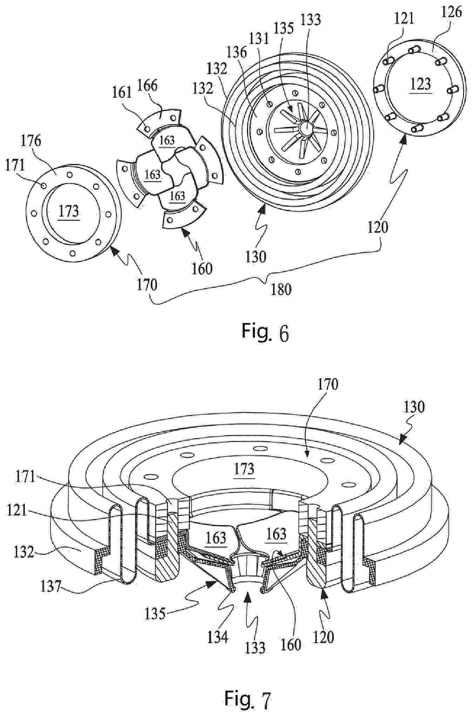

FIG.6-7 illustrate the composition and assembly relationship of said seal membrane assembly 180, which including a lower retainer ring 120, a seal membrane 130, a protection device 160 and an upper retainer ring 170. Said the seal membrane 130 and said protection device 160 are sandwiched between the lower retainer ring 120 and the upper retainer ring 170, moreover, the cylinder 121 of the said lower retainer ring 120 is aligned with corresponding holes on other components in said seal membrane assembly 180. Said cylinder 121 and the bore 171 of the upper retainer ring 170 are adopted to interference fit, so that the whole seal membrane assembly 180 in the compressed state. Said protection device 160 includes 4 protectors 163 arranged so as to protect a center sealing body of said seal membrane 130, herein permit the sharp edge of the instrument to pass through without causing perforations or tears to the seal membrane 130.

Said seal membrane 130 includes a proximal opening 132, a distal end aperture 133, and the sealing wall extending from the distal end to the proximal end, said sealing wall including a proximal surface and a distal surface. Said aperture 133 formed by a sealing lip 134 for accommodating an inserted instrument and forming a gas-tight seal. Said sealing lip 134, in the present embodiment, is approximately circular, but said sealing lip 134 may be non-circular. As described in the background of the invention, the circumference of the sealing lip should be short and strong enough to ensure sealing reliability when a 5mm diameter instrument is inserted. In the present embodiment, the sealing lip 134 is circular, defining its radius as Rlip, so that the circumference of the sealing lip is approximately equal to $2*$ Rlip $*\pi$ ($\pi$=3.14159), usually the circumference of the sealing lip is 11.8~13.8mm. The cross-section of said sealing lip is circular, usually its radius is 0.35 to 0.5 mm diameter.

Said the seal membrane 130 also including the flange 136; The sealing wall 135 has one end connected to the sealing lip 134 and the other end connected to the flange 136; the floating portion 137 has one end connected to the flange 136 and the other end connected to said proximal end 132. Said flange 136 for mounting the protector device 160. Said floating portion 137 including one or several radial (lateral) pleats, so that the entire seal membrane assembly 180 can float in the assembly 200.

Said assembly 180 can be made from a variety of material s with a range of different properties. For instance, said seal membrane 130 is made of a super elastic material such as silicone or polyisoprene; said protector device 160 is made of a semi-rigid thermoplastic elastomer; and said lower retainer ring 120 and said upper retainer ring 170 are made of a relatively hard rigid material such as polycarbonate.

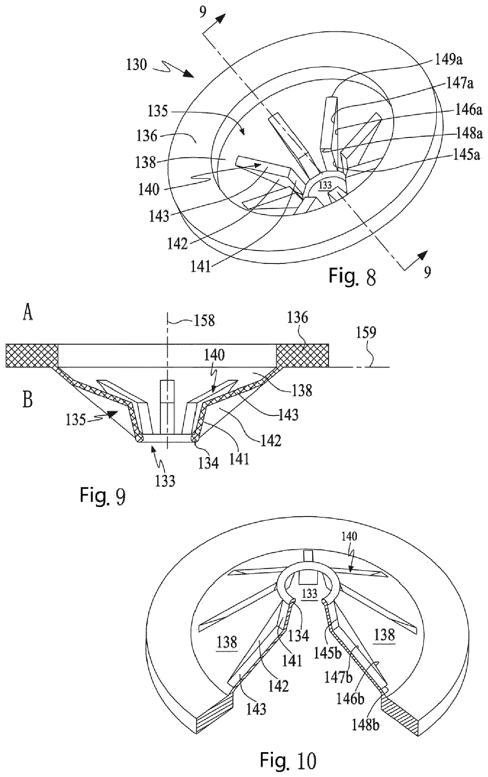

FIG. 8-10 show more detailed depiction of the seal membrane 130 in the first embodiment of the invention. In order to reduce the production cost, the seal membrane 130 is preferably designed as a monolithic part, but can also be designed as an inner seal body and an outer floating portion, separated from the flange 136. The first embodiment is mainly directed to the improvement of the inner seal body. To simplify the description, the outer floating portion and the proximal end are not shown in the subsequent description of the seal membrane.

Said sealing lip 134 comprising a longitudinal axis 158, and a transverse plane 159 that is generally perpendicular to the longitudinal axis 158. Said sealing wall 135, which can be approximately frustum, approximately hemispherical, or an irregularly rotating surface. In this embodiment, said wall 135 is formed in an approximately conical arrangement surrounding the sealing lip 134. Said wall 135 including the main rotary-wall 138 and a plurality of reverse Concave-channels 140 (or hollow convex-rib 140). Said reverse Concave-channel 140 is recessed from the distal surface of the main rotary-wall 138, and the opening of reverse Concave-channel is towards the distal surface; however, in the perspective of proximal surface, said reverse Concave-channel 140 is a hollow convex-rib that is raised from the main rotary-wall 138. Said reverse Concave-channel 140 extends laterally outward from the sealing lip 134, and in the lip-adjacent area, the depth of which gradually increases when the reverse Concave-channel 140 extending laterally outward. The measurement method of the Concave-channel depth is: The shortest distance from the point at the bottom of the Concave-channel recess to the main rotary-wall along longitudinal axis. Said a plurality of reverse Concave-channels 140 divided the main rotary-wall 138 approximately into a plurality of portions, that is said sealing wall 135 is a seamless sealing wall formed by the main rotary-wall 138 and a plurality of reverse Concave-channels 140 arranged around the sealing lip 134 in an approximately conical manner.

Said reverse Concave-channel includes an inner sealing-wall 141, a side sealing-wall 142 and an upper sealing-wall 143. The first edge of said side sealing-wall 142 and said inner sealing-wall 141 formed an intersection line 145a, 145b; the second edge of said side sealing-wall 142 and the main rotary-wall 138 formed an intersection line 146a, 146b; the third edge of said upper sealing-wall 143 and said inner sealing-wall 141 formed an intersection line 148a, 148b; and the other end thereof and the main rotary-wall 138 formed an intersection line 149a, 149b. Defining the angle between said intersection line 146a (146b) and said transverse plane surface 159 as $\alpha$, which is called the guide angle; the angle between said intersection line 145b and said intersection line 146b (or between said intersection line 145a and said intersection line 146a) is defined as $\theta$.

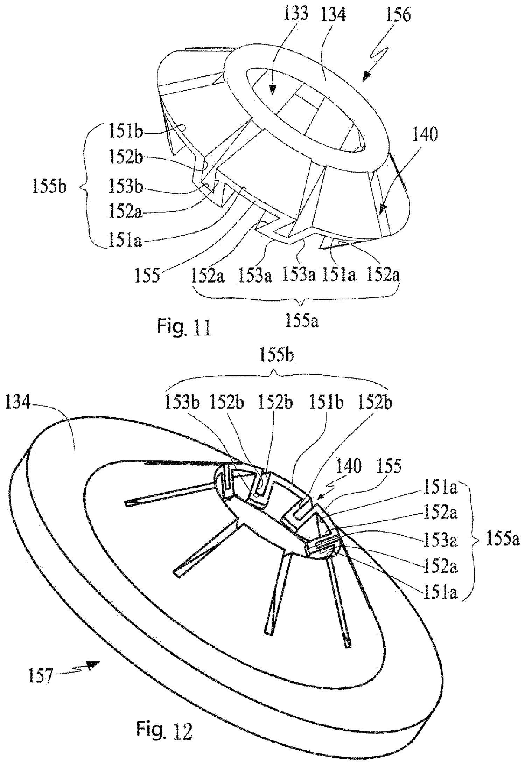

As FIG. 11-11 has shown, in an alternative embodiment, that the thickness of said sealing wall 135 is substantially uniform, that is, the thickness of the main rotary-wall 138, the inner sealing-wall 141, and the side sealing-wall 142 are substantially equal, so that the deformation of the sealing wall 135 is substantially uniform. However, the thickness of said substantially uniform wall should not be limited to the values of the absolute equality. When the number of said Concave-channels is numerous, the thickness of the side sealing-wall 142 can be 0.05~0.25mm thinner than the thickness of the inner sealing-wall 141 (or the main rotary-wall 138) for convenience of manufacture (for example, to enhance the strength of the mold at the Concave-channel), or considering the tolerance. The thickness value of the inner sealing-wall 141, the side sealing-wall 142, and the main rotary-wall 138 is small, for convenience of quantification, the thickness ratio between the inner sidewall 141 (or the main rotary-wall 138) and said side sealing-wall 142 within 1~1.5, which still approximately consider that the thickness of the sealing wall 135 is substantially uniform and still does not deviate from the scope of the invention.

The sealing wall 135, in the present embodiment, comprises eight linear reverse Concave-channels, however, a greater number or a smaller number of non-linear reverse Concave-channel may be adopted. The side sealing-wall 142 of the present embodiment is substantially parallel to the longitudinal axis 158, and in the lip-adjacent area, make a arbitrarily section plane that parallel to said axis 158 and meanwhile perpendicular to side sealing-walls 142, the intersected profile formed by said section plane and said Concave-channels 140 is approximately U-shaped (the intersected profiles of other Concave-channels are also defined in this way). However, for convenience of manufacture, such as mold unloading, said side sealing-walls 142 may not be parallel to the longitudinal axis 158; that is, the section of the reverse Concave-channel 140 is approximately trapezoidal, even approximately V-shaped.

Taking the longitudinal axis 158 as a rotary axis, make a cylindrical surface with a radius R1 and intersects with said main rotary-wall 138 to form an intersection line, and create cutting plane M1 through said intersection line and perpendicular to the generating line of the main rotary-wall 138 (with the axis 158 as rotary axis). Said cutting plane M1 divides the seal membrane 130 into an inner portion 156 (as in FIG. 11) and an outer portion 157 (FIG. 12). Said cutting plane M1 intersects the main rotary-wall 138 to form a plurality of intersection lines 151a and 151b. Said cutting plane M1 intersects the side sealing-wall 142 to form a plurality of intersection lines 152a and 152b, and said cutting plane M1 intersects said inner sealing-wall 141 to form a plurality of intersection lines 153a and 153b.The plurality of segments 151a, 152a, 153a are formed an annular intersection line 155a; the plurality of segments 151b, 152b, 153b are formed an annular intersection line 155b, and the section 155 defined by said annular intersection line 155a and 155b.

As shown in FIG 11-12, it is obvious that the circumference L1 of the intersection line 155a (155b) is much larger than $2*\pi*R1$, that means the reverse Concave-channel plays a role in enlarging hoop circumference, and the difference between L1 and $2*\pi*R1$ is approximately equal to 2*P times the length L2 of the intersection line 153a (153b) (P is the number of reverse Concave-channels). That is, the side sealing-wall 142 actually plays a role in enlarging hoop circumference. With the prerequisite of the reverse Concave-channels width meeting the needs of the manufacturer, increasing the width of the reverse Concave-channel does not mean have a larger hoop circumference.

Those skilled in the art can understand that there must be some R1 value making the outer portion 157, which is divided by the cutting plane M1, to start from the section 155, the main change of its shape is shown as local bending deformation and macroscopic displacement of the seal membrane, rather than the overall microscopic molecular chain elongation and overall tensile deformation. And said inner portion 156, from said sealing lip 134 to said section 155, the change of shape is shown as the comprehensive effect of partial bending deformation and overall tensile deformation of the seal membrane. What it is quite clear is that said reverse Concave-channels enlarge hoop circumference, and reduce the cylinder hoop strain (stress) when a large diameter instrument is inserted, thereby reducing the hoop force and the frictional resistance.

Said side sealing-wall 142 has effects on reinforcing ribs similar to those described in the background, all of the side sealing-walls 142 together reinforcing the axial tensile stiffness in the lip-adjacent area; and said side sealing-walls 142 increase the axial tensile stiffness without increasing the hoop stiffness, thus increasing the axial stiffness without increasing the hoop force, such that which can effectively reduce the stick-slip described in the background. In this example, 16 side sealing-walls 142 are included, while more or less side sealing-walls also can increase the axial tensile stiffness.

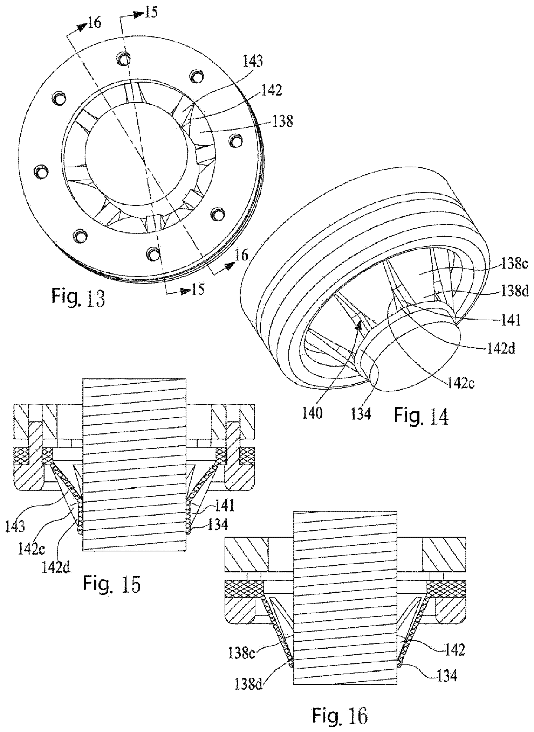

FIG. 13-16 shows a simulated deformation view of s the seal membrane 130 when a large diameter instrument is inserted into said seal membrane assembly 180(the floating portion outside the seal membrane and the protect device 160 are not shown). Said sealing lip 134 is stretched enough to accommodate the inserted instrument, the sealing wall 135 rotates and stretches outwardly around its intersection part with the flange 136. Said inner sealing-wall 141 wrapped outside the instrument; and said side sealing-wall 142 is divided into two portions, a side sealing-wall 142c and a cylindrical wall 142d; said the main rotary-wall is divided into two portions, a conical wall 138c and a cylindrical wall 138d. Said inner sealing-wall 141, said cylindrical wall 142d, and said cylindrical wall 138d together forms the wrapped area around the outer surface of said inserted instrument. Studies have shown that, compared to the grooveless design, the wrapped area of the sealing body with the reverse Concave-channel is small, and the actual contact area of the two surfaces between the instrument and the seal membrane is small, and reducing the wrapped area can reduce the frictional resistance.

In summary, said reverse Concave-channel has the functions of enlarging hoop circumference, reducing the wrapped area, reducing the actual contact area of the two surfaces between the instrument and the seal membrane, increasing the axial tensile stiffness, etc., thereby, the frictional resistance and the stick-slip can be greatly reduced, and the probability of inversion is reduced and the comfort of application is improved.

The stress in the lip-adjacent area, especially said wrapped area is highly concentrated when a large diameter instrument is inserted. It is easy to understand for those skilled in the art that the closer to the sealing lip, the greater the cylinder strain (stress). It has been stated above that the method of increasing the hoop circumference cannot be used to reduce the cylinder strain (stress) of the sealing lip, but the cylinder strain (stress) can be reduced by increasing the hoop circumference in the lip-adjacent area; Moreover, it is necessary to rapidly increase the hoop circumference in the lip-adjacent area so that the cylinder strain (stress)in the lip -adjacent area is rapidly reduced to approximate zero. It has been stated above that said side sealing-wall 142 an important role to enlarge hoop circumference, when said side sealing-wall 142 extending laterally from the sealing lip, the faster the rate of its width increased, the faster the rate of the hoop circumference enlarged in the lip-adjacent area, that is to say, the larger said angle θ is, the faster the rate of the hoop circumference enlarged in the lip-adjacent area. Optionally, the geometry of said reverse Concave-channel 140 is designed to conform to the following equation:

$$\theta \geq \arctan \frac{9\pi R(R_i - R)\cos\alpha}{P(R_i + 9R)(R - R_0)}$$

and α+θ≤90°

Where:

θ=the angel between two edges of the reverse Concave-channel side sealing-wall in the lip-adjacent area

α=the angle between the rotary generating line and the transverse plane surface in the lip-adjacent area

arctan= arctangent function

cos = cosine function

π=circumference ratio

R=radius

Ri=the largest radius designed for the surgical instrument passed through the seal membrane

R0=radius of the sealing lip

P=number of reverse Concave-channel

A reasonable value of θ make the hoop circumference increased rapidly in the lip -adjacent area. According to the above equation, normally, Ri and R0 is constant; The variables α, P and R together affect the rate of hoop circumference enlarged. Normally selecting 0°≤α≤50°, by theoretical analysis and related research, it is shown that reducing the value of the guiding angle α is advantageous for reducing the length of said wrapped area. However, too small a guide angle α will sacrifice the guiding performance of the seal membrane, so it is necessary to base on the premise of satisfying the guiding performance when determining the value of α as small as possible. Normally 2.5mm≤R≤ (Ri+ R0)/2. If the value of R is less than 2.5mm, the transition area at the sealing lip will be too large; if the value of R is greater than (Ri+ R0)/2, the effect of enlarging the hoop circumference in the lip-adjacent area and reducing the hoop force is not obvious. Reasonable values of α, P and R can ensure good guiding and minimize the wrapped area when a large diameter instrument is inserted, and the hoop force in the lip -adjacent area is rapidly reduced to a small value, or disappeared. It has been found that R=3.5, P=8, α=35° can ensure good guiding, and reduce the wrapped area and the hoop force. Approximate or possibly more efficient combinations of parameters can be obtained by theoretical calculations and simple experimental verification.

The design of said reverse Concave-channel, as long as it conforms to the essence of the above equation, should be considered as be substantially coincident with the above equation. For instance, those skilled in the art readily conceive that said main rotary-wall 138, said inner sealing wall 141 or said side sealing-wall 142 is designed as non-linear curved surface; or deliberately designing the main rotary-wall 138, the inner sealing wall 141 or the side sealing-wall 142 into a complex multi-faceted surface; the intersection line may not be a straight line, as long as the two intersection lines are viewed as a whole, the angle between the adjacent area of the sealing lip substantially will conforms with the above equation, it can be considered that the scope of the present invention is not deviated.

The previous part has been described in detail the lip-adjacent area as an area with high concentration of stress, said strain (stress) outside herein relatively small. As long as said reverse Concave-channel in the lip -adjacent area substantially conforms to above equation, it is not necessary if the reverse Concave-channel outside the lip-adjacent area conforms to the above equation or not. In this example, outside the lip-adjacent area, said side sealing-wall 142 is a surface defined by two sides extending laterally outward and gradually narrowing; that is, the depth of the reverse Concave-channel 140 is rapidly decreased outside the lip-adjacent area, which does not conform to the above equation. Those skilled in the art will understand that such design of reverse Concave-channel not only ensure that the reverse Concave-channel has the functions of enlarging the hoop circumference, reducing the wrapped area and the two surfaces in contact between the instrument and the seal membrane, and increasing the axial tensile stiffness, etc. ,but also greatly simplify mould design, improve the efficiency of the seal membrane processing, and help to reduce the actual contact area of the two surfaces between the instrument and the seal membrane..

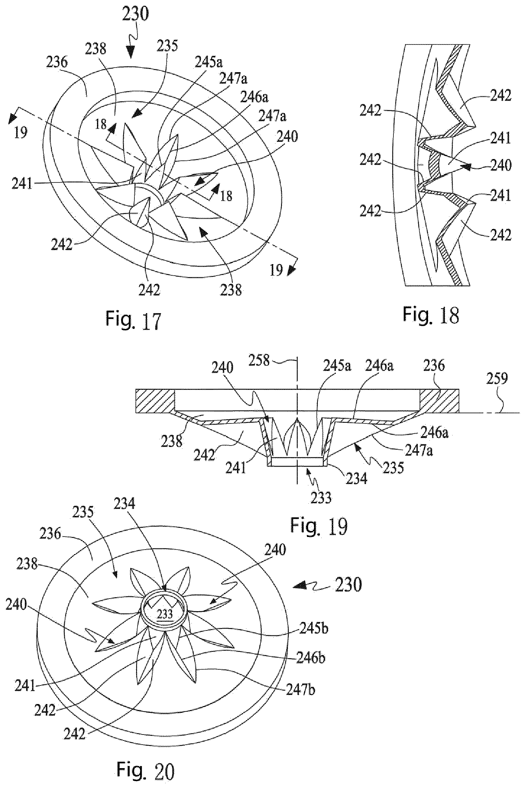

FIG. 17-20 illustrate a second embodiment of the seal membrane 230 of the invention. Said seal membrane 230 includes a distal aperture 233, a sealing lip 234, a sealing wall 235 and a flange 236, said distal aperture 233 formed by the sealing lip 234, said sealing wall 235 connecting the sealing lip 234 at one end and the flange 236 at the other end, said the seal membrane including the proximal surface and the distal surface, the sealing lip 234 comprising a longitude axis 258, and a transverse plane 259 substantially perpendicular to the longitude axis 258.

In this embodiment, said sealing wall 235 is formed in an approximately conical arrangement around the sealing lip 234. Said sealing wall 235 includes the main rotary-wall 238 and a plurality of reverse Concave-channel 240 (or hollow convex-rib 240). Said reverse Concave-channel 240 is recessed from the distal surface of the main rotary-wall 238 pointing in the direction towards the proximal end, and the opening of reverse Concave-channel is towards the distal surface; however, in the perspective of proximal surface, said reverse Concave-channel 240 is a hollow convex-rib that is raised from the main rotary-wall 238. Said reverse Concave-channel 240 extends laterally from the sealing lip 234 in the direction away from the longitudinal axis 258. Within the lip-adjacent area, the depth of said reverse Concave-channel 240 gradually increases along the axial direction of the sealing lip; and the depth of reverse Concave-channel 240 gradually decreases outside the lip-adjacent area, said a plurality of reverse Concave-channels 240 divided the main rotary-wall 238 approximately into a plurality of portions. That is, the sealing wall 235 is a seamless sealing wall formed by the main rotary-wall 238 and a plurality of reverse Concave-channels 240 arranged around the sealing lip 234 in an approximately conical manner. The sealing wall 235, in the present embodiment, comprises 8 linear reverse Concave-channels, however, a more or less number of non-linear reverse Concave-channels may be adopted.

Said reverse Concave-channel includes an inner sealing-wall 240 and a side sealing-wall 242. The first edge of said side sealing-wall 242 and said inner sealing-wall 241 are formed an intersection line 245a, 245b; two adjacent side sealing-wall 242 are formed an intersection line 246a,246b; said side sealing-wall 242 and the main rotary-wall 238 are formed the intersection line 2467a, 247b. Defining the angle between said intersection line 247a(247b) and said transverse plane surface 259 as κ, which is called the guide angle κ.

The seal membrane 230 with reverse Concave-channels has the similar functions to the seal membrane 130, including enlarging the hoop circumference, reducing the wrapped area, reducing the two surfaces in contact between the instrument and the seal membrane, increasing the axial tensile stiffness, etc.

The main difference between said seal membrane 230 and the seal membrane 130 is the sectional shape of the reverse Concave-channel 240 is approximately triangular; however, the sectional shape of the reverse Concave-channel 140 is approximately U-shaped. Referring to Figures 17-20, draw the longitudinal section 16-16 of the seal membrane 230 through the intersection lines 246a and 246b; draw the projection line 245e of the intersection line 245a in the section 16-16 (not shown);and draw the projection line 247e of the intersection line 247a in the section 16-16 (not shown). Defining the angle between the projection line 245e and the intersection line 247e as $\lambda$. Alternatively, the angle $\lambda$ and the guiding angle $\kappa$ also approximate the aforementioned equation of $\theta$ angle, so that the reverse Concave-channel increases the hoop circumference faster. (i.e. $\lambda$ is substituted for $\theta$ in the aforementioned equation , and $\kappa$ is substituted for $\alpha$ in the aforementioned equation).

It will be understood by those skilled in the art, under the premise of the same dimension, that the U-shaped reverse Concave-channel has a greater ability to increase the hoop circumference than the V-shaped reverse Concave-channel. However, the V-shaped reverse Concave-channel is simpler and more economical to manufacture and more productive than the U-shaped.

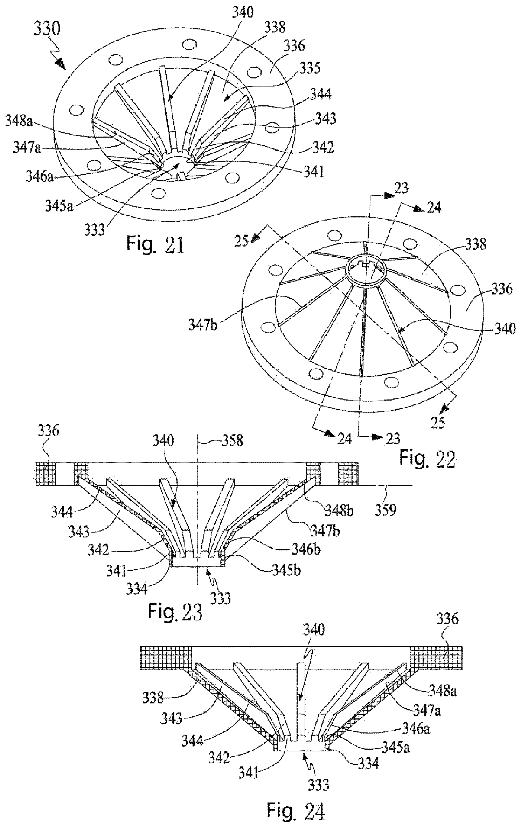

FIG.21-24 illustrate the third embodiment of the seal membrane 330 of the present invention. Said seal membrane 330 includes a distal aperture 333, a sealing lip 334, a sealing wall 335 and a flange 336, said distal aperture 333 formed by the sealing lip 334, said sealing wall 335 connecting the sealing lip 334 at one end and the flange 336 at the other end. Said the seal membrane 330 includes the proximal surface and the distal surface, the sealing lip 334 comprising a longitude axis 358, and a transverse plane 359 substantially perpendicular to the longitude axis 358.

In this embodiment, the sealing wall 335 is formed in an approximately conical arrangement surrounding the sealing lip 334. Said sealing wall 335 including the main rotary-wall 338 and a plurality of reverse Concave-channels 340 (or hollow convex-rib 340). Said reverse Concave-channels 340 are recessed from the distal surface of the main rotary-wall 338 pointing in the direction towards the proximal end, and the opening of reverse Concave-channel is towards the distal surface; while in the perspective of proximal surface, said reverse Concave-channel 340 is a hollow convex-rib that is raised from the main rotary-wall 338. Said reverse Concave-channel 340 extends laterally from the sealing lip 334 to the longitudinal axis 358, and in the lip-adjacent area, the depth of said reverse Concave-channel 340 gradually increases along the axial direction of the sealing lip. Said a plurality of reverse Concave-channels 340 are divided the main rotary-wall 338 approximately into a plurality of portions, that is, the sealing wall 335 is a seamless sealing wall formed by the main rotary-wall 338 and a plurality of reverse Concave-channels 340 arranged around the sealing lip 334 in an approximately conical manner. The sealing wall 335, in the present embodiment, comprises 10 linear reverse Concave-channels, however, a more or less number of non-linear reverse Concave-channel may be adopted.

Said reverse Concave-channel 340 includes an inner sealing-wall 341, an inner sealing-wall 342, a side sealing-wall 343, and an upper sealing-wall 344. Said inner sealing-wall 341 connects the inner sealing-wall 342 and the side sealing-wall 343 to the sealing lip 334. Said side sealing-wall 343 and said inner sealing-wall 341 are formed an intersection line 345a, 345b; said side sealing-wall 343 and said inner sealing-wall 342 are formed an intersection line 346a, 346b; said side sealing-wall 343 and the main rotary-wall 338 are formed the intersection line347a, 347b; The side sealing-walls 343 and the upper sealing-walls 344 are formed the intersection lines 348a, 348b. Defining the angle between said intersection line 347a(347b) and said transverse plane surface 159 as $\upsilon$, which is called the guide angle $\upsilon$.

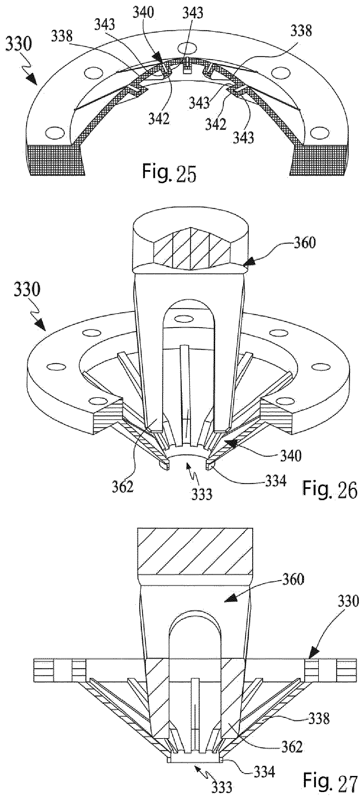

FIG.25 illustrates, in an alternative embodiment, the overall thickness of the reverse Concave-channel 340 is much thinner than which of the main rotary-wall 238, and that is, the thickness of the inner sealing-wall 341, the inner sealing-wall 342, and the side sealing-wall 343 are much thinner than which of the main rotary-wall 338. The inner width B1 of the hollow convex-rib 240 (or the reverse Concave-channel 240) measured in the lip-adjacent area from the distal surface should be as small as possible. The thickness T1 of said side sealing-wall 343 is much smaller than the thickness T2 of the main rotary-wall 338. For convenience of manufacture, normally, $0.25mm \leq T1 \leq 0.45mm$; and $0.6mm \leq T2 \leq 1.5mm$. Increasing the thickness of the seal membrane can enhance the tear resistance thereof but at the same time, the hoop force must be increased; reducing the thickness of the seal membrane can reduce the hoop force, but at the same time, the seal membrane is inevitably damaged.

Those skilled in the art will understand that titanium applier is one of the instruments most prone to damage the seal membrane, when the minimally invasive surgical instrument passes through the trocar. When the applier is operated, its cutting edge is opened to form a U-shaped fork structure. When the seal membrane is thin, the working edge of the applier easily penetrates into the contact between the seal membrane and the working edge and forces the contact point to be recessed, thereby stretching and piercing it. When the seal membrane is thick, it is difficult for the working blade to force the contact point to be recessed, and the blade against the surface of the seal membrane slides to the center hole of the seal membrane, thereby avoiding the seal membrane to be punctured. It will be understood by those skilled in the art that when the inner wall thickness of the reverse Concave-channel 340 is small (i.e., the inner sealing-wall 341, the inner sealing-wall 342, the side sealing-wall 343 is thin), especially the reverse Concave-channel when the inner width of the reverse Concave-channel 340 is small, the stiffness of the reverse Concave-channel 340 against compression resistant is enhanced. Moreover, since the reverse Concave-channel 340 is convex relative to the main rotary-wall 138, when the reverse Concave-channel is deformed by pressure, said reverse Concave-channel 340 will be curved on its side instead of being recessed. Referring to FIGS. 26-27, when the titanium applier 360 is inserted, the working edge 362 of said applier 360 is bent toward the reverse Concave-channel 340, the reverse Concave-channel 340 is curved and deformed so that said working blade slides toward the main rotary-wall 338; and the main rotary-wall 338 is relatively thick and is not easily crushed to be recessed and stretched to be pierced, and the working edge 362 is relatively slid with the surface of the main rotary-wall 338, and is slid toward the distal aperture 333.

The use of the reverse Concave-channel with a thinner wall is propitious to reducing said frictional resistance by reducing the hoop force; the use of a thicker main rotary-wall can prevent the seal membrane from being damaged by the inserted instrument. That is, the reasonable value of T1 and T2 can greatly improve the durability of the seal membrane without sacrificing the comfort of operation. The actual values of T1 and T2 can be obtained through simple test verification. Moreover, the reverse Concave-channel 340 of the present example also has similar functions as the aforementioned reverse Concave-channel 140.

The sealing lip 334 of said seal membrane 330 is cylindrical and there is a transition zone composed of a plurality of inner sealing-walls 341. Said transition zone will increase the contact area between the inserted instrument and the sealing membrane and increases the cylindrical wrapped area when a large diameter instrument is inserted. Therefore, the axial height HI of the inner sealing-wall 341 in the transition zone should be smaller. The value of the axial height HI should generally be smaller than half the distance from the sealing lip to the boundary of the lip-adjacent area. When the axial height HI is smaller, the influence of the inner sealing-wall 341 can be ignored when analyzing the reverse Concave-channel. When the axial height H1 is larger, the influence of the inner sealing-wall 341 can be ignored when analyzing the reverse Concave-channel.

Alternatively, the axial height HI is smaller, and the intersection line 346b intersects the intersection line 347b at a point in the inner region of the sealing lip 334. Defining the angle between the intersection line 346b and the intersection line 347b as $\beta$, moreover, the influence of the inner sealing-wall 341 on said reverse Concave-channel 340 is ignored. Said angle $\beta$ and the guiding angle $\upsilon$ also are conformed to approximate the aforementioned equation of angle $\theta$, so that the reverse Concave-channel increases the hoop circumference faster. (i.e. $\beta$ is substituted for $\theta$ in the aforementioned equation, and $\upsilon$ is substituted for $\alpha$ in the aforementioned equation).

Alternatively, if the axial height HI of the inner sealing-wall 341 is larger, the function of the inner sealing-wall 341 will be similar to that of the inner sidewall 141, and in this state, the angle between intersection line 345b and intersection line 347b is defined as $\gamma$. Said angle $\beta$ and the guiding angle $\upsilon$ also approximate the aforementioned equation of angle $\theta$, so that the reverse Concave-channel increases the hoop circumference faster.(i.e. $\gamma$ is substituted for $\theta$ in the aforementioned equation, and $\upsilon$ is substituted for $\alpha$ in the aforementioned equation).

Those skilled in the art easily understand that said reverse Concave-channel is a relative concept. Taking the seal membrane 130 for an example, taking the upper sealing-wall 143 of the reverse Concave-channel 140 as a reference, the two adjacent side sealing-walls 142 and the main rotary-wall 138 which between the walls will form the sunken Concave-channel relative to the upper sealing-wall 143 (referred to as front Concave-channel). Comparing the area of the upper sealing-wall 143 with the main body rotating wall 138, the size is different, the shape of the Concave-channel is obviously different. The different shapes of the Concave-channels produce different effects for the wrapped area and the actual contact area is also different of the instrument and the seal membrane. Taking the seal membrane 130 for an example, the projected area of the main rotary-wall 138 on the transverse plane 159 is defined as A1; the projected area of all reverse Concave-channels 140 on the transverse plane 159 is defined as A2. In order to more clearly define the scope of the present invention, it is defined that when A2 < A1, said Concave-channel 140 is a "reverse" Concave-channel defined by the present invention; when A2 $\geq$ A1, the Concave-channel 140 is not the "reverse" Concave-channel defined by the invention.

Those skilled in the art easily understand that the reasonable fillet transition can avoid stress concentration or make certain areas deformed more easily. Due to the small size of the seal membrane, especially the area near the sealing lip is smaller, with such a small size and different chamfer, the shape of the seal membrane looks different. In order to clearly show the geometric relationship of the elements, the embodiment of the invention is generally the pattern without the fillet.

Many different embodiments and examples of the invention have been shown and described. One of those ordinary skilled in the art will be able to make adaptations to the methods and apparatus by appropriate modifications without departing from the scope of the invention. The structure and the manner of fixing of the protector assembly disclosed in US7788861 are used in the example of the present invention. However, the structure and the manner of fixing of the protector assembly disclosed in US7798671 can be used, and in some applications, the protector assembly may not be included. The approximate U-shaped Concave-channel and the approximate V-shaped described in this embodiment cannot be limited to U-shaped or V-shaped. It has been mentioned many times in the invention that the Concave-channel extends laterally outward from the sealing lip, and the so-called "extending laterally outward" should not be limited to a straight line. Said "extending laterally outward" can be a spiral, a line segment, a multi-section arc line and so on. In the invention, the positional relationship of the intersecting surfaces composed of said Concave-channel and the intersection line thereof are described with reference to specific embodiments, and the methods of increasing curved surfaces to form a multifaceted mosaic or using of the high-order curved surface to make the intersection line and the Concave-channel shape to look different from said embodiment. However, it can be considered not deviated from the scope of the invention, as long as it conforms to the general idea of the invention. Several modifications have been mentioned, to those skilled in the art, other modifications are also conceivable. Therefore, the scope of the invention should follow the additional claims, and at the same time, it should not be understood that it is limited by the specification of the structure, material or behavior illustrated and documented in the description and drawings.

**Claims**

1. A trocar seal membrane for minimally invasive surgery, comprises a proximal opening, a distal aperture, and a sealing wall which extends from the distal aperture to the proximal opening, said the sealing wall comprising a proximal surface and a distal surface, said distal aperture formed by a sealing lip for accommodating the inserted instrument and forming a gas-tight seal, said sealing lip comprising a longitudinal axis and a transverse plane substantially perpendicular to said longitudinal axis; wherein:

   a) said sealing wall comprises the main rotary wall and a plurality of reverse Concave-channels, which is recessed from the distal surface of the main rotary wall toward the proximal surface and the reverse Concave-channel opening oriented to the distal surface, the shape of the reverse Concave-channel from the perspective of proximal surface is represented as a rib that is raised from the proximal face;

   b) in thelip-adjacent area, each of the reverse Concave-channels comprises two side walls, said side walls are areas defined by the two edges extending laterally outward from the sealing lip and with a gradually increasing width.

2. The seal membrane of claim 1, wherein said main rotary wall is frustum, in thelip-adjacent area, a plurality of reverse Concave-channels substantially dividing the main rotary wall into a plurality of portions; the projected area of said main rotary wall on said transverse plane is defined as A1; the projected area of said reverse Concave-channel on said transverse plane is defined as A2, and A2 < A1; said reverse Concave-channel has the functions: enlarging the hoop circumference, reducing the wrapped area, reducing the actual contact area of the two surfaces between the instrument and the seal membrane and increasing the axial tensile stiffness.

3. The seal membrane of claim 1, wherein the cross section of said reverse Concave-channel is approximately U-shaped or V-shaped, or one portion of it is U-shaped and the other portion is V-shaped.

4. The seal membrane of claim 1, wherein the angle between two sides of said side wall conforms to the following equation:

$$\theta \geq \arctan \frac{9\pi R(R_i - R)\cos\alpha}{P(R_i + 9R)(R - R_0)}$$

and $\alpha + \theta \leq 90°$ ;
Where:

   $\theta$= the angel between two sides of the reverse Concave-channel side wall in the lip-adjacent area
   $\alpha$ = The angle between the rotary generating line and the transverse plane surfacein the lip-adjacent area
   arctan= arctan function
   cos = cosine function
   $\pi$=circumference ratio
   R=radius
   Ri=the largest radius designed for the surgical instrument passed through the seal membrane $R_0$=radius of the sealing lip
   P=number of reverse Concave-channel.

5. The seal membrane of claim 3, wherein the thickness of the reverse Concave-channel is smaller than the thickness of the main rotary wall.

6. The seal membrane of claim 1, wherein the seal membrane also includes a flange which and said main rotary wall extend to be intersected or simultaneously the main rotary wall and the reverse Concave-channel extend to be intersected, and an outer floating portion including at least one lateral pleat extending from the flange to the proximal opening proximal opening.

7. A trocar seal assembly, wherein the seal membrane comprises any one of said seal membranes as defined in claims 1 to 5, and including a lower retainer ring, a upper retainer ring, a protection device, a upper body and a upper cover; said seal membrane further includes a flange and said main rotary wall extend to be intersected or simultaneously the main rotary wall and the reverse Concave-channel extend to be intersected, and an outer floating portion including a plurality of lateral pleats extending from the flange to the proximal opening; said seal membrane and said protect device are sandwiched between the upper retainer ring and the lower retainer ring, said proximal opening are sandwiched between the upper body and the upper cover.

723

700

720
721
730
750
710
711
713

Prior art

Fig.1

Di
741
737
732
730
736
735
738
734 733
742
ANG1

Fig. 2 Prior art

735c
738a
735d

Prior art Fig. 3

721

735g
738f
735e

Fig. 4 Prior art

Fig. 5

EP 3 494 910 A1

Fig. 6

Fig. 7

17

Fig. 8

Fig. 9

Fig. 10

133  134  156

151b
152b
155b  153b
152a
151a

155

155  152a  153a  153a  151a  152a

140

Fig. 11

155a

155b

134  153b  152b  152b  151b  152b

140  155

151a
152a
153a  155a
152a
151a

157

Fig. 12

Fig. 13

Fig. 14

Fig. 15

Fig. 16

Fig. 17

Fig. 18

Fig. 19

Fig. 20

Fig. 21

Fig. 22

Fig. 23

Fig. 24

Fig. 25

Fig. 26

Fig. 27

# INTERNATIONAL SEARCH REPORT

International application No.

**PCT/CN2017/093605**

## A. CLASSIFICATION OF SUBJECT MATTER

A61B 17/34 (2006.01) i

According to International Patent Classification (IPC) or to both national classification and IPC

## B. FIELDS SEARCHED

Minimum documentation searched (classification system followed by classification symbols)

A61B 17/-

Documentation searched other than minimum documentation to the extent that such documents are included in the fields searched

Electronic data base consulted during the international search (name of data base and, where practicable, search terms used)

CNPAT, WPI, EPODOC, CNKI: airproof, instrument, wave, ridge, sinew, skull patch, varus, reverse, puncture, trocar, seal, film, membrane, slot, fold, wrinkle, pleat.

## C. DOCUMENTS CONSIDERED TO BE RELEVANT

| Category* | Citation of document, with indication, where appropriate, of the relevant passages | Relevant to claim No. |
|---|---|---|
| PX | CN 106037899 A (CHENGDU WUYI MEDICAL TECHNOLOGY CO., LTD.), 26 October 2016 (26.10.2016), claims 1-7, description, paragraphs [0040], [0049] and [0069], and figures 1-27 | 1-7 |
| X | CN 101478924 A (ETHICON ENDO-SURGERY, INC.), 08 July 2009 (08.07.2009), description, page 2, line 12 to page 6, line 2, and figures 1-5 | 1, 3-7 |
| A | CN 101474089 A (ZHOU, Xing et al.), 08 July 2009 (08.07.2009), the whole document | 1-7 |
| A | CN 204428125 U (CHANGZHOU WECARE MEDICAL TECHNOLOGY CO., LTD.), 01 July 2015 (01.07.2015), the whole document | 1-7 |
| A | CN 103169528 A (ZHEJIANG GEYI MEDICAL INSTRUMENT CO., LTD.), 26 June 2013 (26.06.2013), the whole document | 1-7 |
| A | US 2011040255 A1 (AESCULAP AG.), 17 February 2011 (17.02.2011), the whole document | 1-7 |

☐ Further documents are listed in the continuation of Box C.    ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 09 October 2017 (09.10.2017) | **18 October 2017 (18.10.2017)** |

| Name and mailing address of the ISA/CN: <br> State Intellectual Property Office of the P. R. China <br> No. 6, Xitucheng Road, Jimenqiao <br> Haidian District, Beijing 100088, China <br> Facsimile No.: (86-10) 62019451 | Authorized officer <br><br> **CHEN, Cheng** <br><br> Telephone No.: (86-10) **52745020** |

Form PCT/ISA/210 (second sheet) (July 2009)

## INTERNATIONAL SEARCH REPORT
### Information on patent family members

| International application No. |
| --- |
| **PCT/CN2017/093605** |

| Patent Documents referred in the Report | Publication Date | Patent Family | Publication Date |
| --- | --- | --- | --- |
| CN 106037899 A | 26 October 2016 | None | |
| CN 101478924 A | 08 July 2009 | BR PI0711570 A2 | 08 November 2011 |
| | | AU 2007237921 B2 | 31 May 2012 |
| | | RU 2455954 C2 | 20 July 2012 |
| | | US 2007255218 A1 | 01 November 2007 |
| | | EP 2010072 A1 | 07 January 2009 |
| | | KR 20090021337 A | 03 March 2009 |
| | | RU 2008145495 A | 27 May 2010 |
| | | WO 2007121425 A1 | 25 October 2007 |
| | | US 7789861 B2 | 07 September 2010 |
| | | AU 2007237921 A1 | 25 October 2007 |
| | | KR 101325945 B1 | 07 November 2013 |
| | | JP 5184513 B2 | 17 April 2013 |
| | | JP 2009534124 A | 24 September 2009 |
| | | CN 101478924 B | 09 November 2011 |
| CN 101474089 A | 08 July 2009 | CN 101474089 B | 29 December 2010 |
| CN 204428125 U | 01 July 2015 | None | |
| CN 103169528 A | 26 June 2013 | None | |
| US 2011040255 A1 | 17 February 2011 | JP 2009531100 A | 03 September 2009 |
| | | ES 2350505 T3 | 24 January 2011 |
| | | US 2009082735 A1 | 26 March 2009 |
| | | EP 1998692 B1 | 13 October 2010 |
| | | JP 4961471 B2 | 27 June 2012 |
| | | EP 2329775 A2 | 08 June 2011 |
| | | ES 2415510 T3 | 25 July 2013 |
| | | AT 484245 T | 15 October 2010 |
| | | US 7842014 B2 | 30 November 2010 |
| | | DE 502007005342 D1 | 25 November 2010 |
| | | EP 1998692 A1 | 10 December 2008 |
| | | WO 2007110371 A1 | 04 October 2007 |
| | | EP 2481367 A1 | 01 August 2012 |
| | | EP 2329775 B1 | 15 May 2013 |
| | | DE 102006015690 A1 | 11 October 2007 |

Form PCT/ISA/210 (patent family annex) (July 2009)

**EP 3 494 910 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7112185 B
- US 7591802 B
- US 5342315 A
- US 5827228 A
- EP 0994740 A
- US 7842014 B
- CN 101480354 A
- US 7788861 B
- US 7798671 B